# EUROPEAN PATENT SPECIFICATION

(11) **EP 4 003 294 B1**
(45) Date of publication and mention of the grant of the patent: **01.07.2026**
(21) Application number: 20751318.5
(22) Date of filing: 29.07.2020
(51) Int. Cl.: A61K 9/00, A61K 9/51, A61K 36/752, C11B 9/00

(54) **NANOTECHNOLOGY-BASED DELIVERY SYSTEM OF BERGAMOT ESSENTIAL OIL, METHOD OF PREPARATION OF THE SYSTEM AND USES THEREOF**
NANOTECHNOLOGIEBASIERTES ABGABESYSTEM FÜR ÄTHERISCHES BERGAMOTTEÖL, VERFAHREN ZUR HERSTELLUNG DES SYSTEMS UND VERWENDUNGEN DAVON
SYSTÈME D'ADMINISTRATION D'HUILE ESSENTIELLE DE BERGAMOTE, FONDÉ SUR UNE NANOTECHNOLOGIE, PROCÉDÉ DE PRÉPARATION DU SYSTÈME ET UTILISATIONS CORRESPONDANTES

(30) Priority: 30.07.2019 IT 201900013353
(43) Date of publication of application: 01.06.2022
(73) Proprietor: Universita' Della Calabria, 87036 Arcavacata di Rende (CS) (IT); Istituto Sant'Anna Di Ezio Pugliese S.r.l., 88900 Crotone (KR) (IT); DZNE ( The German Center for Neurodegenerative Diseases), 53127 Bonn (DE)
(72) Inventor: RUSSO, Rossella, 87036 ARCAVACATA DI RENDE (CS) (IT); MIZOGUCHI, Hirokazu, Sendai Miyagi, Miyagi 981-8558 (JP); WATANABE, Chizuko, Sendai Miyagi, Miyagi 981-8558 (JP); HAMAMURA, Kengo, Fukuoka, 815-8511 (JP); KATSUYAMA, Soh, Saitama 362-0806 (JP); KOMATSU, Takaaki, Fukuoka, Fukuoka 815-8511 (JP); MORRONE, Luigi Antonio, 87036 ARCAVACATA DI RENDE (CS) (IT); ADORNETTO, Annagrazia, 87036 ARCAVACATA DI RENDE (CS) (IT); LAGANA', Annarita Stella, 87036 ARCAVACATA DI RENDE (CS) (IT); SCUTERI, Damiana, 87036 ARCAVACATA DI RENDE (CS) (IT); CORASANITI, Maria Tiziana, 88100 CATANZARO (CZ) (IT); TONIN, Paolo, 88900 Crotone (KR) (IT); SAKURADA, Shinobu, Sendai, Miyagi, Miyagi 981-8558 (JP); SAKURADA, Tsukasa, Fukuoka, Fukuoka 815-8511 (JP); NICOTERA, Pierluigi, 53177 Bonn (DE); BAGETTA, Giacinto, 87036 ARCAVACATA DI RENDE (CS) (IT); CASSANO, Roberta, 87036 ARCAVACATA DI RENDE (CS) (IT); TROMBINO, Sonia, 87036 ARCAVACATA DI RENDE (CS) (IT); ROMBOLA', Laura, 87036 ARCAVACATA DI RENDE (CS) (IT)
(74) Representative: Gitto, Serena
(86) International application number: PCT/IT2020/050189
(87) International publication number: WO 2021/019588

(56) References cited:
- WO-A2-2011/116963
- US-A- 5 856 361
- BAGETTA G ET AL: "Neuropharmacology of the essential oil of bergamot", FITOTERAPIA, IDB HOLDING, MILAN, IT, vol. 81, no. 6, 1 September 2010 (2010-09-01), pages 453 - 461, XP027172546, ISSN: 0367-326X, [retrieved on 20100723]

## Description

The present invention concerns a nanotechnology-based delivery system of bergamot essential oil (BEO), method of preparation of the system and uses thereof, as defined by the claims, In particular, the present invention concerns α-tocopheryl stearate- Solid Lipid Nanostructures (α-TFS-SLNs) loaded with bergamot essential oil without psoralens (BEO-BF).

It is well known that essential oils are plant extracts that have a history of popular use as aromatherapeutic agents for a large range of properties depending on the source of the extract.

In particular, bergamot essential oil is widely used for its properties. The bergamot plant, i.e. *Citrus bergamia Risso et Poiteau,* belongs to the family *Rutaceae.*

*Citrus bergamia* is defined as a hybrid of bitter orange (*Citrus aurantium* L.) and lemon (*Citrus limon* L.) Burm. Fil., by some authors, or of *Citrus aurantium* L. and *Citrus aurantifolia* (Christm.) Swing by others (Rapisarda & Germanò, 2013). It is mainly cultivated for its essential oil that is obtained by rasping and cold pressing the fruit peel.

Bergamot essential oil (BEO) is a greenish or brownish-yellow volatile oil with a bitter aromatic taste and a characteristic pleasant odor that made bergamot popular in cosmetics in the past and especially in aromatherapy in our days. BEO is included in the official Pharmacopoeias of various countries. According to the *Official Italian Pharmacopoeia* (12th Ed.), BEO is obtained by cold pressing of the epicarp and, partly, of the mesocarp of the fresh fruit. Percentages of more characteristic components in bergamot essential oil (Farmacopea Ufficiale Italiana, 12th Ed.) are reported in the Table 1.

**Table 1**

| Chemical substance | Interval ranges (%) |
|---|---|
| α-Pinene | 0.7-2.0 |
| Sabinene | 0.5-2.0 |
| β-Pinene | 5.0-10.0 |
| Limonene | 30.0-50.0 |
| γ-Terpinene | 6.0-18.5 |
| Linalool | 6.0-15.0 |
| Linalyl acetate | 23.0-35.0 |
| Geranial | < 0.5 |
| Geranyl acetate | 0.1-0.7 |
| Cariophyllene | 0.2-0.5 |

BEO comprises a volatile fraction (93-96% of total) containing monoterpene and sesquiterpene hydrocarbons (such as limonene, α- and β-pinene, β-myrcene, γ-terpinene, terpinolene, sabinene and β-bisabolene); oxygenated derivatives (such as linalool, neral, geranial, linalyl acetate, neryl acetate and geranyl acetate); and a nonvolatile fraction (4-7% of total) containing waxes, polymethoxylated flavones, coumarins and psoralens such as bergapten (5-methoxypsoralen) and bergamottine (5-geranyloxypsoralen) (Mondello et al., 1993; Dugo et al., 2000).

Oxygenated compounds, namely linalool and linalyl acetate, mark the flavor notes of BEO.

The hydrocarbon fraction does not have a fundamental role in determining the olfactory character of BEO. The nonvolatile residue is a natural odor fixative which influences the olfactory properties of the oil; however, it contains about 0.2% bergapten which is responsible for the phototoxicity of BEO (Zaynoun et al., 1977; Ashwood-Smith et al., 1980). Therefore, a bergapten-free essential oil (BEO-BF) together with an essential oil deprived of the hydrocarbon fraction and of bergapten (BEO-HF/BF) are prepared by extractive industries for perfumery and cosmetic uses (see Bagetta et al., 2010).

BEO is widely used by the cosmetic industries (e.g., in perfumes, body and sun tanning lotions, soaps) for its intense fragrance and freshness, being main ingredient of eau-de-cologne. The International Fragrance Association recommends a maximum of 0.4% bergamot oil in the final products for application to areas of skin exposed to sunshine. In order to obtain safer products, bergapten has to be removed or reduced to a level of 15 ppm in the final product (Costa et al., 2010).

In the pharmaceutical industries BEO is used as a flavouring for some medicinal products and as a cicatrizing, for its antiseptic and antibacterial proprieties while it is also used in aromatherapy.

Historically, BEO has been used in Italian folk medicine since 1725, primarily for fever and worms. Empirical observations on the antimicrobial properties and wound healing effects of BEO were first published in 1800 by Francesco Calabrò, a physician from Reggio Calabria (see Focà & Liberto, 2015). BEO has been used for mouth, skin, respiratory and urinary tract infections, gonococcal infections, leucorrhoea, vaginal pruritus. Traditional therapeutic uses (see Focà & Liberto, 2015) for preparations of bergamot essential oil are:
a) liquid antiseptic for medical uses, local infections, personal hygiene;
b) solid disinfectant for daily hygiene;
c) ointment for disinfection of nasal cavities and mouth; and
d) preparation intended to be converted into vapor for the treatment of respiratory diseases.

In Hungary, a registered "healing product" in form of ointment containing a mixture of herbal preparations, including BEO, has been on the market since 2008 for mitigation of symptoms (erythema, infiltration, paraceratosism, urticaria) and for nursing of dry, pealing, squamous skin in the mild or moderate psoriasis.

In industrialized countries, likewise other essential oils, BEO is widely used in aromatherapy, and has recently received renewed popularity to improve mood and mild symptoms of stress-induced disorders such as anxiety, depression, behavioural disturbances in dementia and chronic pain (Bagetta et., 2010; Chang & Shen, 2011; Ndao et al., 2012; Ni et al., 2013; Morrone et al., 2017; Scuteri et al., 2017).

Currently, about 35 million people all over the world are affected with dementia, of which Alzheimer's Disease (AD) represents the most widespread form(WHO, 2012; Achterberg et al., 2013).

Apart from cognitive disturbances and memory loss, about the 20% of patients suffering from AD living in the community (Lyketsos et al., 2000; Ballard et al., 2013) and the 40-60% of the nursing home residents (Margallo-Lana et al., 2001; Ballard et al., 2013) are also affected by the Behavioral and Psychological Symptoms of Dementia (BPSDs), in particular agitation and aggression (Ballard et al., 2013). The BPSDs remarkably reduce the individuals' health-related quality of life (HRQL), almost as the cognitive deficits do. BPSDs affect four domains: Behavior (with aggression, agitation and sleep disorders), Mood (with depression, anxiety and mania), Thought Content (with delusions and illogical thoughts) and Perception (with hallucinations and misidentification) (Burns et al., 1990a; 1990b; 1990c; 1990d; Huang et al., 2012). Almost all the demented patients develop at least one of these symptoms during the whole evolution of dementia (Tariot et al., 1995). A multicentre longitudinal prospective study, the Medical Research Council Cognitive Function and Ageing Study (MRC CFAS), highlighted the co-occurrence of anxiety and depression and the association of psychotic symptoms with apathy (Savva et al., 2009).

Since AD is widespread among the elderly, it is likely that they suffer from arthritis, responsible of chronic osteoarticular pain, or from diabetes or herpes zoster, which cause neuropathic pain. Moreover, these patients can experience breakthrough pain, likely because of cancer. Pain is tightly involved in the development of the BPSDs (in particular agitation and aggression) (Corbett et al., 2012) and neuropathological changes involving the matrix of modulation of pain perception and of integration of the emotional and behavioural manifestations of pain may assume the key importance in the development of BPSDs.

Apart from the presence of comorbidity, responsible for the development of chronic pain syndromes and impairment of painful inputs perception and integration, the poor communication capabilities of demented patients deserve particular attention. These patients find noteworthy difficulty in conveying pain, thus developing BPSDs such as agitation and aggression.

The crucial role of chronic pain in the development of BPSDs can be demonstrated also by the evidence that even a complete and accurate adherence to the therapy of the cognitive decline can delay the onset of psychological symptoms (even though without specific benefit for agitation and aggression) (Ballard et al., 2013) and provides some improvements in the prevention and general handling of BPSDs (Ballard et al., 2013), but it does not definitely prevent the patients from experiencing them (Ballard et al., 2013).

Currently, the pharmacological treatment of the BPSDs is represented by the atypical antipsychotics risperidone, olanzapine, aripiprazole and quetiapine. These drugs, with better evidence for risperidone in particular, result safer and effective for a short-term period of 6-12 weeks and in aggression mainly rather than in agitation (Ballard et al., 2006; Schneider et al., 2006; Ballard et al., 2009; Scuteri et al., 2017).

The lack of efficacy on agitation could be due to the chronic pain component on which these pharmacological agents do not act. Indeed, the effect of pain treatment on the control of the BPSDs has been examined with a multicenter cluster randomized controlled trial (ClinicalTrials.gov NCT01021696 and Norwegian Medicines Agency EudraCTnr 2008-007490-20), carried out in 60 Norwegian nursing homes from October 2009 to June 2010 on demented patients of age≥65 years (Husebo et al., 2011). The control group received the usual treatment, while the patients belonging to the intervention group received painkillers, following a standardized stepwise protocol (oral paracetamol, oral morphine, buprenorphine transdermal patch or oral pregabalin). Indeed, opioids represent one of the most used class of analgesics, even if there is not wide evidence to support their effectiveness for chronic non cancer pain (Morrone et al., 2017). The intervention group presented a significant average reduction of 17% in the primary outcome that was agitation, measured by the Cohen Mansfield Agitation Inventory, with respect to the control group. Furthermore, an increased agitation score was detected when the analgesic drugs were withdrawn (Husebo et al., 2011). Therefore, according to this study, the treatment of pain states is important for the control of agitation in demented patients, in order to employ the atypical antipsychotics only when it becomes unavoidable, thus reducing the risk of serious side effects and of increased mortality associated with the use of antipsychotics(Scuteri et al., 2017).

Interestingly, aromatherapy with *Melissa* and *Lavandula officinalis* has obtained the best results for the treatment of agitation in patients affected with AD (Ballard et al., 2009). The patients suffering from dementia frequently present dysfunctional olfaction (Jimbo et al., 2009) and neurofibrillary tangles in the entorhinal cortex, already in the early stages (Braak et al., 1991; Gold et al., 2000; Jimbo et al., 2009). Therefore, there is a tight link between olfaction and AD and the aromatic molecules, binding to olfactory epithelium acceptors, can induce psychological responses. However, for pharmacological effects to occur systemic absorption of the active principles is needed to trigger the release of soluble cell mediators. In particular, the mechanism of action of both *Melissa officinalis* and *Lavandula officinalis* may involve the cholinergic system (Carnat et al., 1998; Perry et al., 1998; 1999; Hohmann et al., 1999; Wake et al., 2000; Petersen et al., 2003; Kennedy et al., 2003; Barocelli et al., 2004; Dastmalchi et al., 2009). This characteristic, shared with the acetylcholinesterase inhibitors used for the cognitive impairment in AD, supports the utility of these two aromatic plants for the management of the BPSDs.

Aromatherapy with *Melissa officinalis* and *Lavandula officinalis* does not definitely solve the problem of agitation and aggression, likely because the antinociceptive effect of the former aromatic plants is weak.

Patients suffering from AD experience pain states, of chronic nature mainly, such as neuropathies. Recently, it was found that neuropathic pain is linked to an impairment of autophagy (Berliocchi et al., 2011); abnormalities of this constitutive process of organelles and proteins turn over have been implicated in AD as well (Yu et al., 2005). Autophagy has been found to undergo different alterations according to the considered neuropathic pain model (Berliocchi et al., 2015).

BEO has been demonstrated to enhance both basal and induced autophagy, with experiments carried out in human SH-SY5Y neuroblastoma cells (Russo et al., 2014). Behavioural studies have pointed out that BEO is able to reduce mechanical allodynia in neuropathic pain models (Bagetta et al., 2010; Kuwahata et al., 2013). Thus, aromatherapy using an essential oil endowed with significant analgesic properties, such as BEO, could result much more effective in the treatment of BPSDs.

Indeed, the growing interest in aromatherapy has prompted great basic research effort in order to propose molecular mechanisms that can account for the behavioural effects of the essential oils, as the whole phytocomplex or the single components, even though further rigorous scientific studies are still necessary. In particular, the behavioural effects of increased locomotor and exploratory activity of rat fostered by BEO have been correlated to increased energy in discrete frequency bands of the electroencephalographic (EEG) spectrum, thus demonstrating that BEO is associated with quantifiable and reproducible effects on gross behaviour and EEG spectrum power (Rombola et al., 2009). Furthermore, BEO has been demonstrated *in vitro* and *in vivo* studies to be able to induce a modulation of the synaptic levels of amino acid neurotransmitters in the rat central nervous system (Morrone et al., 2007). These findings explain the behavioural effects of BEO. Indeed, glutamatergic neurons could be involved in behaviour and nociception (Manns et al., 2003; Bagetta et al., 2010); metabotropic glutamate receptors in hippocampus may play an important role in the establishment of coherent beta rhythm that could potentiate synaptic transmission, with consequent involvement in sensory processing and cognitive function (Boddeke et al., 1997; Bagetta et al., 2010).

However, exocytosis and receptor mediated neurotransmitter release are such fundamental mechanisms that modulation described for BEO may concern any neurotransmitter, including endogenous opioid peptides, in the central and peripheral nerve tissue.

Accordingly, these mechanisms might be highly relevant to the development of pain, either nociceptive or neuropathic, and to its treatment. In fact, strong evidence has been gathered for BEO to be endowed with analgesic activity, both in nociceptive and in neuropathic pain models. Thus, intraplantar (i.pl.) BEO, or its components linalool and linalyl acetate, reduced the nociceptive response as assayed by the capsaicin test (Sakurada et al., 2011). The latter antinociceptive effects were antagonized by the ipsilateral intraplantar (i.pl.) administration of naloxone hydrochloride and by intraperitoneal (i.p.) naloxone methiodide, an antagonist acting at the peripheral, but not at the central nervous system (CNS), opioid receptors. Morphine-induced antinociception after i.p. and intrathecal (i.t.) administration was markedly enhanced by the combined injection of i.pl. BEO or linalool, its main oxygenated monoterpene (Sakurada et al., 2011). Interestingly, for i.pl. injection BEO or linalool reduced partial sciatic nerve ligation (PSNL)-induced neuropathic pain symptoms in mice and inhibition of spinal ERK phosphorylation seems to be involved in this anti-allodynic effect (Kuwahata et al., 2013). These same Authors (Sakurada et al., 2011) have shown that BEO or linalool modulate morphine-induced anti-allodynic effect under neuropathic pain, a condition known to be resistant to opioid treatment (see Arner et al., 1988). Furtherly, the antinociceptive properties of BEO were examined in the formalin test (Katsuyama et al., 2015). The formalin test consisted in the i.pl. administration of 20 µl of formalin at 2% (diluted in a saline solution), which determined a behavioural response of licking/biting, characterized by a first phase (lasting for about 10 minutes [min] after formalin injection) and a late phase (from 10 to 30 min following the administration of the formalin solution). Likely, the first phase has a peripheral origin, while the second phase is characterized by a central nature. BEO or Jojoba wax, as control, were administered in pretreatment 10 min before formalin: BEO resulted effective in both phases of the formalin test, only when injected into the ipsilateral hindpaw, thus confirming the peripheral nature of the antinociceptive effect (Katsuyama et al., 2015). The pretreatment with the opioid receptor antagonist naloxone methiodide, which does not cross blood brain barrier, produced an attenuation of the effect of BEO on the nociceptive behaviour. Therefore, the data gathered so far suggest the involvement of peripheral opioid receptors in the antinociceptive activity of BEO (Katsuyama et al., 2015).

On the basis of the above, bergamot essential oil can overcome the disadvantages of *Melissa officinalis* and *Lavandula officinalis* based aromatherapy, since it has a stronger antinociceptive effect and, therefore, it could be advantageously used for aromatherapy, in particular in complementary medicine.

However, at present, aromatherapy is carried out by addition of an aromatic material to massage oil, bath oil, candle wax, pillow stuffing, or to a device such as a vaporizer or a diffuser. These delivery systems of aromatherapy do not allow the exposure of individuals to known amount of the phytocomplex or of principal ingredients to be established. Accordingly, dose-effect relationships in clinical settings for any of the biological, therapeutic or side effect cannot be performed thus hampering the reproducibility of the therapeutic effect.

In addition, it is known that some volatile compounds, in particular monoterpenes such as limonene and pinene, are relatively unstable in heat and light, they are rapidly oxidized and undergo hydration reactions and structural rearrangements. In fact, according to the prior art, it is necessary to protect these unstable compounds in order to increase the product's shelf life (Reverchon & lacuzio 1997; Fang 2004).

Therefore, in the clinical setting of the above cited delivery systems, together with the light and chemical instability of aromas, do not allow the amount of the active phytocomplex or ingredients the individual is exposed to be established precisely and, hence, do not warrant reproducibility of the aromatherapeutic response.

Accordingly, efficacy and safety studies in clinical settings for any of the aromatherapeutic effect cannot be performed, thus hampering their reproducibility. In fact, apart from the psychological effects, known to be short-lived and mostly depending on the association of the latter effects with previous olfactory exposure of the subject, aromatherapeutic effects of essential oils are to be attributed to pharmacological mechanisms implicating interaction between active ingredients of the phytocomplex with receptors expressed in the site of action.

In the light of the above, it is therefore apparent the need to provide a delivery system of BEO able to overcome the disadvantages of the known delivery system of BEO.

The delivering of BEO phytocomplex (without psoralens) in a known amount, increasing stability of the compounds, and therefore product's shelf-life, represent the aim of this invention in order to obtain an effective aromatherapy, such as in complementary medicine, with reproducibile aromatherapeutic effects.

According to the present invention a nanotechnology BEO delivery system has now been found, as defined by the claims, which is able to deliver known amounts of the phytocomplex, without psoralens (BEO-BF), and to protect its ingredients, in particular volatile compounds, from chemical instability and from light or heat inactivation, increasing the phytocomplex shelf life.

Therefore, according to the present invention, it is now possible to effectively use aromatherapy with BEO-BF in complementary medicine for the therapy of acute and chronic pain and prevention or treatment of BPSDs and any other stress, including itch-related behavioural and mood disorder in normal or demented people.

In particular, previously devised solid lipid nanostructures (SLN), such as those disclosed in the patent US5250236 (1993), were modified to incorporate an antioxidant agent (Trombino et al., 2009). In particular, the solid lipid nanoparticles disclosed in US5250236 do not contain any antioxidant agent in their matrix, while the solid lipid nanoparticles disclosed in Trombino et al., 2009, contain ferulic acid as anti-oxidant agent. WO 2011/116963 A2 discloses stabilized solid lipid nanoparticles comprising active compounds, which are stabilized by a further outer polymeric coating. The actives can be selected from divers cosmetic and pharmaceutical compounds which include i.a. essential oils

According to the present invention, an ester obtained by the reaction between alpha-tocopherol, i.e. vitamin E, and stearic acid has been used as anti-oxidant agent, which provides unexpected advantageous results.

In fact, it was surprisingly found that the modified SLN according to the present invention are able to prolong the shelf life of the phytocomplex and ingredients therein.

Moreover, an important advantage of the nanotechnology BEO delivery system according to the present invention consists of a longer lasting physical and chemical stability of the phytocomplex, whose constant strength in biologically active components is mandatory for reproducibility of aromatherapeutic effects.

The nanotechnology BEO delivery system according to the present invention presents also the advantage of being odorless, i.e. it does not exhale the smell of BEO.

In addition, to facilitate the application of BEO-BF-SLNs on the skin, a dermatological formulation, based of SLN loaded BEO-BF, was prepared without a heat-based method, thus avoiding the degradation of BEO-BF and the destruction of the SLN.

The nanotechnology-based delivery system for aromatherapy with BEO-BF according to the present invention, and in particular the dermatological preparation for skin application in the form of cream, i.e. a nanocream delivery system (NDS), can be advantageously used in complementary medicine, in particular to complement the therapy of acute and chronic pain and prevention or treatment of BPSDs and any other stress (including itch) related behavioural and mood disorders in normal or demented people.

The results obtained testing the nanotechnology BEO delivery system according to the present invention showed stable and reproducible aromatherapy effects. In particular, after two and six months of light exposure the active ingredients' amounts declined only by 10% and 18%, with no further degradation at twelve months.

Therefore, the advantage of this delivery system is the prolonged physicochemical stability (up to 12 months) of the phytocomplex and of its main components allowing reproducible antinociceptive and anti-itch responses to be measured.

As mentioned above, the nanotechnology BEO delivery system according to the present invention can be administered in the form of cream, for skin application. The main advantage of the use of the delivery system of the present invention as carrier for the transdermic release is the significant reduction of the total loss of transepidermic water.

In addition, the nanoparticles according to the present invention are characterized by a large surface area which allows the prolonged exposure and contact of the loaded BEO with the skin, resulting in an efficient release.

Furthermore, aromatherapy is basically devoid of serious adverse drug reactions and its efficacy allows side effects of painkillers to be minimized. In particular, according to the World Health Organization (WHO) guidelines for the stepwise use of painkillers, the invention may allow the use of any painkillers to be delayed and their therapeutic effects and those of opioids against acute or chronic pain, from moderate to severe grade, to be enhanced and, hence, their doses and side effects to be reduced. Better pain control under complementary aromatherapy with the invention may prevent or cure BPSDs known to occur in a large proportion of demented people as consequence of unreported pain currently treated with antipsychotic drugs devoid of analgesic properties but endowed with serious adverse drug reactions responsible for doubling the risk of death.

It is therefore specific object of the present invention solid lipid nanoparticles, wherein said nanoparticles are α-tocopheryl stearate based and comprise bergamot essential oil free of psoralens.

The solid lipid nanoparticles according to the present invention have a size of 444.03nm ± 60.07 (mean ± S.D.), whereas the polydispersity index is approximately 0.30.

The bergamot essential oil according to the present invention comprises limonene, linalool and linalyl acetate.

The invention concerns also a pharmaceutical composition comprising solid lipid nanoparticles as defined above, as active principle, in association with one or more excipients and/or adjuvants.

The pharmaceutical composition can be in the form of a cream or gel.

A further object of the present invention is solid lipid nanoparticles as defined above or pharmaceutical composition as defined above for use as a medicament.

According to an embodiment, the present invention concerns the solid lipid nanoparticles as defined above or the pharmaceutical composition as defined above, for use in the treatment of acute and chronic pain, such as the neuropathic type of pain or pain occurring in the course of cancer or pain occurring in the course of chronic neurodegenerative diseases, such as Parkinson's disease and Alzheimer's disease.

According to a further embodiment, the present invention concerns the solid lipid nanoparticles as defined above or the pharmaceutical composition as defined above, for use in the prevention or treatment of behavioural and psychiatric symptoms of dementia, or of behavioural or mood disturbances emerging from stressful conditions, such as itch.

The present invention concerns also a method for obtaining solid lipid nanoparticles as defined above, said method comprising or consisting of:
a) mixing α-tocopheryl stearate in the presence of bergamot essential oil, for example bergamot essential oil in a percentage of 10% in comparison to the amount of α-tocopheryl stearate, by heating, in particular at a temperature lower than temperatures that degrade bergamot essential oil, preferably in the range of 60-65 °C; b) adding a water solution of a one or more emulsifiers, such as polysorbate 20, polysorbate 60, soy phosphatidylcholine, and sodium taurodeoxycholate, preferably sodium taurodeoxycholate, one or more co-emulsifiers, such as sodium monooctylphosphate, n-butanol, preferably butanol, water (Advanced Drug Delivery Reviews 47 (2001) 165-196; Chemistry and Physics of Lipids174 (2013) 32-38; Indian J. Pharm. Sci., 2009, 71 (4): 349-358), in order to obtain a optically transparent microemulsion; c) dispersing the microemulsion in cold water under agitation, preferably in a ratio microemulsion:cold water of 1:20. Preferably, the temperature of cold water is about 2°C, whereas the agitation is preferably performed by high-speed homogenation or mechanical stirrer. Magnetic agitation furnishes higher polydispersity of the nanoparticles preferably high-speed homogenation.

The present invention now will be described by an illustrative, but not limitative way, according to preferred embodiments thereof, with particular reference to the enclosed drawings, wherein:

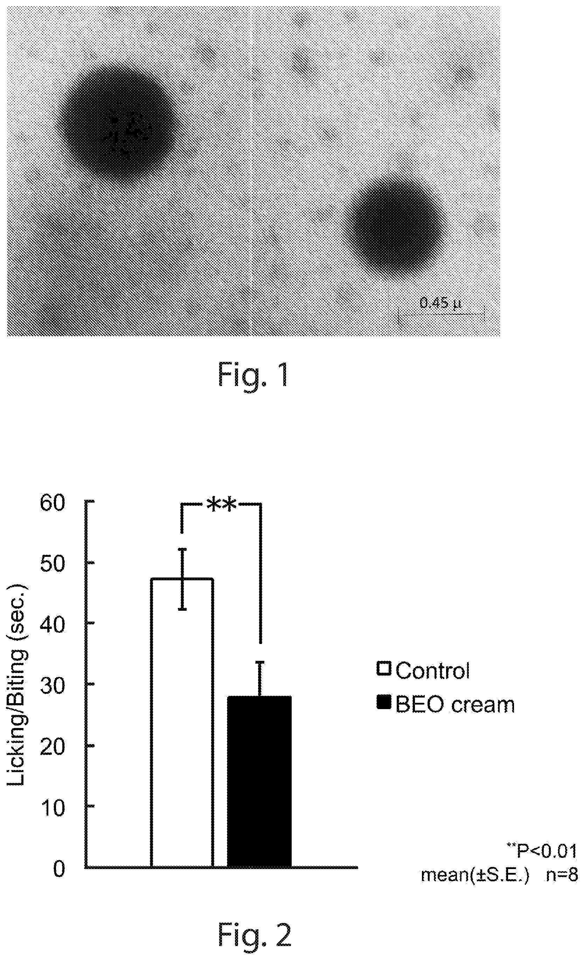

Figure 1 shows Photomicrography of SLNs by Trasmission Electron Microscopy. Ultrastructure of the modified Solid Lipid Nanoparticles (SLN) loaded with bergamot essential oil without psoralens (BEO-BF) is shown. At six months the percentage of linalool, linalyl acetate and limonene SLNs containing was declined of 17.7+2.52% respect to that present into SLNs analysed at time zero (linalool=10.6 mg; linalyl acetate=12.1 mg and limonene=14.1 mg, respectively).
Figure 2 shows the Effects of NDS with BEO-BF on capsacin-induced nociceptive behavior in mice. In particular, it is shown that, using the lot n° 1/2016 of the pharmaceutical cream preparation, BEO-BF encapsulated into modified SLN (cream) NDS is endowed with antinociceptive properties in the capsaicin test in mice. NDS BEO-BF cream (1 mg) (test) or empty (control) (1mg) were applied to the plantar surface of the right hindpaw in mice (n= 8) 30 min before capsaicin injection and cumulative time (sec) spent in licking/biting behaviour was monitored for a 5 min session.

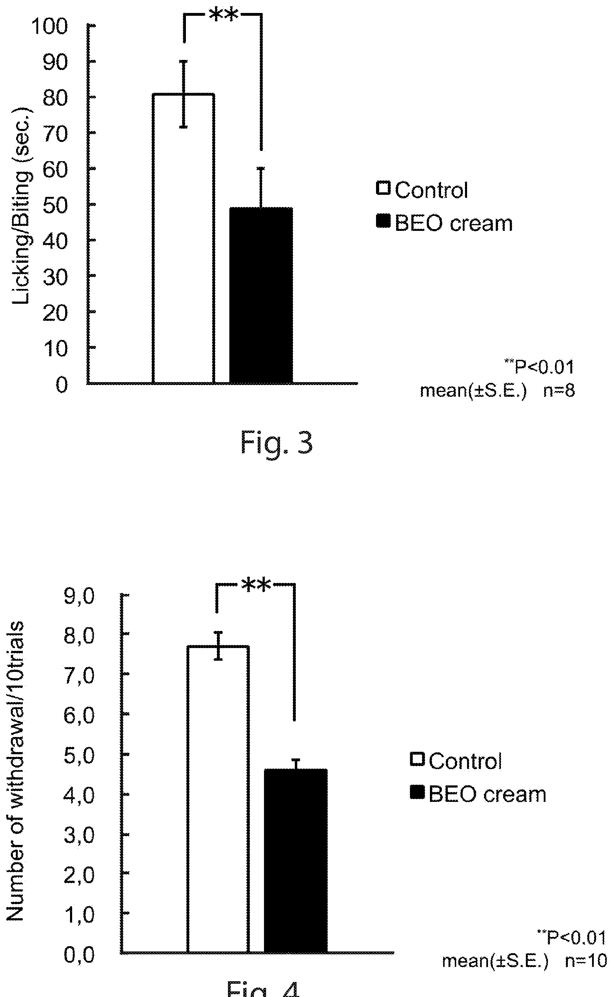

Figure 3 shows the Effects of NDS with BEO-BF on formalin-induced nociceptive behavior in mice. In particular, it is shown that, using the lot n° 1/2016 of the pharmaceutical cream preparation, BEO-BF encapsulated into modified SLN (cream) NDS is endowed with antinociceptive properties in the formalin test in mice. NDS BEO-BF cream (1 mg) (test) or empty (control) (1 mg) were applied to the plantar surface of the right hindpaw in mice (n= 8) 30 min before formalin test and cumulative time (sec) spent in licking/biting behaviour was monitored for a 30 min session.
Figure 4 shows the Effects of NDS with BEO-BF on neuropathic pain in mice with partial sciatic nerve ligation. In particular, it is shown that, using the lot n° 1/2016 of the pharmaceutical cream preparation, BEO-BF encapsulated into modified SLN (cream) NDS is endowed with antiallodynic properties in the partial sciatic nerve ligation (PSNL) test model in mice. NDS BEO-BF cream (1 mg) (test) or empty (control) NDS cream (1mg) were applied to the plantar surface of the right hindpaw in mice (n=10) 30 min before sensitization measurement. BEO (test) and control have been administered on day 7 after surgery and mechanical allodynia has been measured as withdrawals of the foot upon application of the Von Frey filament (3.0 g)and repeated in ten sessions (modified from Kuwahata et al., 2013).

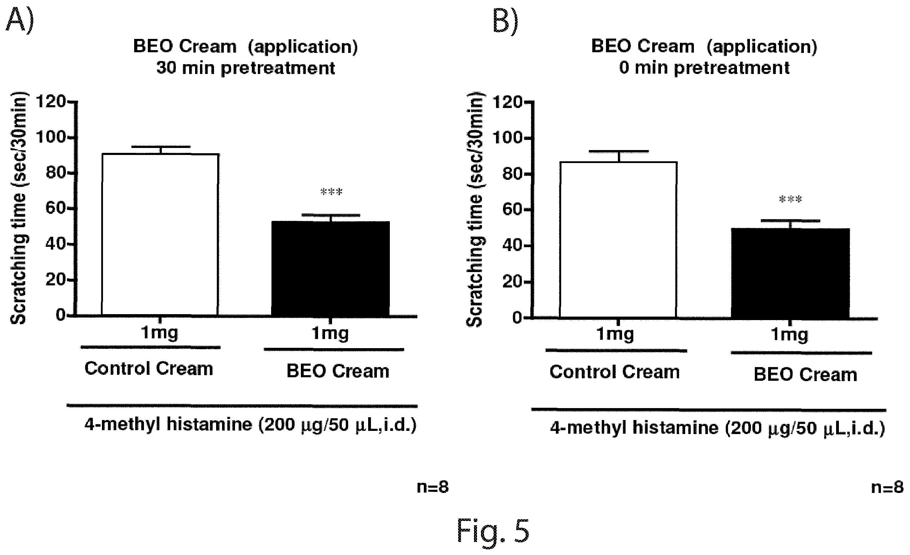

Figure 5 shows the Effects of BEO cream on itching in mice. In particular, it is shown that, using the lot n° 1/2016 of the pharmaceutical cream preparation, BEO-BF encapsulated into modified SLN (cream) NDS is endowed with anti-itching properties in the test model provided by intradermal (i.d.) administration of 4 methyl-histamine (200 µg/50 µl, i.d.) in rear region of the neck of mice. NDS BEO-BF cream (1 mg) (test) or empty NDS cream (1mg) (control) were applied on the shaved skin of the rear region of the neck 30 min before(Fig.5A; n= 8 mice) or immediately (0 min) before (Fig.5B; n=8 mice) 4 methyl-histamine intradermal (i.d) administration in the shaved skin region and scratching behaviour was measured for 30 min following administration.

### EXAMPLE 1: Preparation of α-TFS-SLNs and related cream for skin application.

### Reagents

All solvents were of analytical grade and purchased from Sigma-Aldrich (Sigma Chemical Co.,St. Louis, MO, USA): tetrahydrofuran (THF), chloroform (CHCl3), n-hexane, ethyl acetate, dimethyl sulphoxide (DMSO), isooctane, 1-butanol, α-linolenic acid (PM = 278.43 g/mol), α-tocopherol (PM = 430.72 g/mol), biliary salt of the taurodeossicolic acid, Tween 20, dicyclohexylcarbonymide (DCC).

### Synthesis of alfa-tocopheryl stearate (α-TFS)

α-tocopheryl stearate (α-TFS) was synthesized according to the procedure described in the literature (Taniguchi et al., 1998). Briefly α-tocopherol (1.0g, 5.15 mmol), p-toluensulfonic acid (0.07g, 0.34mmol) and stearyl alcohol (0.7g, 5.4mmol) were added to 10ml dry toluene under stirring and N₂ at room temperature. When the addition was complete, the solution was stirred under N₂ for 12h at 110°C. After cooling at room temperature, the solvent was evaporated under reduced pressure, the residue was treated with 15ml water and the aqueous phase was extracted with chloroform (4×15ml). The combined organic phases were collected and dried using natrium sulphate (Na₂SO₄), a common inorganic drying agent that acquire water of hydration when exposed to moist air or a wet solution. Afterwards the solvent was removed by rotary evaporation to give yellow-colored alpha tocopheryl stearate (α-TFS) and purified by Merck silica gel (60-230 mesh) column chromatography, using ethylacetate/n-hexane85/15(v/v) as eluent. The solvent of eluated ester was evaporated under reduced pressure. The α-TFS was identified by TLC, UV and 1H-NMR analyses (Bernards and Lewis, 1992; Kawanishi et al.,1990; Taniguchi et al., 1998).

### Preparation of α-TFS-SLNs

α-tocopheryl stearate - Solid Lipid Nanoparticles (α-TFS-SLNs) were prepared by a microemulsion technique at moderate temperature (Gasco, 1997). In particular, α-tocopheryl stearate-Solid Lipid Nanoparticles (α-TFS-SLNs) were prepared by a microemulsion technique. Briefly, α-tocopheryl stearate (α-TFS, 142 mg, 0,201 mmol) in the absence and/or in the presence of BEO (*National origin: Italy*) (40 mg, 10% of α-TFS, 0.0201 mmol) was mixed at a temperature ranging from 60 to 65°C. A warm water (0.21 mg, 6% of α-TFS 0.0012 mmol) solution of sodium taurodeoxycholate (25.33 mg, 30% α-TFS, 0.061 mmol), butanol (0.3 mg, 2% of α-TFS, 0.0041 mmol) and Tween 20 (75 mg, 30% of α-TFS, 0.061 mmol) was then added to obtain an optically transparent system. Sodium taurodeoxycholate and Tween 20 act as emulsifiers and butanol as co-emulsifier. The warm microemulsion was immediately dispersed in cold water (2°C) under high-speed homogenation (Model SL2, Silverson, Chesham Bucks, England) at 8000 rpm for 30 min (240.000 g in 30 min). The volume ratio of warm microemulsion to cold water was 1:20. The α-TFS-SLN dispersions were washed twice using an Amicon TCF2A ultrafiltration system (Amicon Grace, Beverley, MA USA; membrane Amicon Diaflo YM 100).

### Size distribution analysis

The size of the α-TFS-SLNs was determined by dynamic light scattering (DLLS) using a 90 Plus Particle Size Analyzer (Brookhaven Instruments Corporation, NewYork, USA) at 25°C by measuring the autocorrelation function at 90° scattering angle. Cells were filled with 100 µl of sample solution and diluted to 4ml with filtered (0.22 µm) water. The polydispersity index (PI) indicating the measure of the distribution of nanoparticle population was also determined (Koppel, 1972). The SLNs showed a diameter equal to 450 nm and a polydispersity index of 0.30.

### Morphology of SLN

The morphology of the hydrated SLN dispersions was examined using TEM. In particular, a drop of SLN dispersion was applied to a carbon-coated copper grid and left for 1 min to allow some of the particles to adhere to the carbon substrate. The excess of dispersion was removed by adsorbing the drop with a piece of filter paper. A drop of 1% phosphotungstic acid solution was applied and again excess of solution was removed by adsorbing the liquid with the tip of a filter paper and the sample was air-dried. The sample was then observed under a ZEISS EM 900 electron microscope at an accelerating voltage of 80 kV. The image observed is shown in Figure 1.

### Percentage of BEO-BF incorporated into α-TFS-SLN

α-TFS-SLN formulation (1ml) was diluted to 10 ml with methanol and, to evaluate BEO-BF content, was analyzed by spectrophotometric detection at wavelengths of:
281 nm for Linalol,
208 nm for a Linalyl acetate, and
247 nm for Limonene, respectively.

The results indicated the presence, in α-TFS-SLN of:
27 % of Linalol,
31% of Linalyl acetate, and
36% of Limonene.

After two and six months of light exposure the above percentages declined by 10% and 18%, respectively, with no further degradation at twelve months, demonstrating that α-TFS-SLNs effectively slow down degradation of BEO-BF.

### Preparation of a nanocream delivery system (NDS) based on α-TFS-SLN containing BEO-BF (44.227 g)

The composition of cream based on α-TFS-SLN containing BEO-BF is reported below:
- 37.604 g of purified water suspension of α-TFS-SLN containing BEO-BF,
- 4.42 g of sweet almond oil,
- 0.885 g of polyacrylamide,
- 0.442 g of isoparaffin C13-14,
- 0.111g of 7-laurate
- 0.774 g of purified water Ph.Eur.,
- 0.028 g of methyl paraben, and
- 0.009 g of propylparaben.

### EXAMPLE 2: Prolonged antinociceptive and anti-itching actions of the BEO-BF encapsulated in NDS cream shown on animal experimental models.

### Capsaicin test

NDS BEO-BF cream (1 mg) or empty NDS cream (1mg) were applied to the plantar surface of the right hindpaw of a mice 30 min before capsaicin injection.

In these experiments, male ddY (SD) mice (Shizuoka Laboratory Center, Japan) weighing 22-26 g, at the time of testing, were used. The mice were individually housed in a colony maintained in a controlled environment (12 h light/dark cycle, room temperature 23 °C, 50-60% relative humidity). The animals had free access to food pellets and water. All behavioural experiments took place during the light period between 10:00 and 17:00 h in a quiet room. The animals were tested in randomized order.

Antinociception was assessed using the capsaicin test as previously described in Sakurada et al. (1992) (Sakurada et al., 1992). To reduce variability, each mouse was acclimatized to an acrylic observation chamber (22.0 × 15.0 × 12.5 cm) for approximately 1 h before the injection of capsaicin. The mouse was injected 20 µl of a solution of capsaicin (0.8 µg/paw) beneath the skin of the plantar surface of the right hindpaw using a 50 µl Hamilton microsyringe with a 26-gauge needle as quickly as possible, with only minimal animal restraint. Following capsaicin injection, the animals (n=16) were immediately placed in the test box for a 5-min observation period. Licking/biting behaviour induced by intraplantar injection of capsaicin was observed as an indicator of nociceptive response. The accumulated response time in second spent in licking/biting the capsaicin-injected paw was measured for a period of 5 min immediately after subcutaneous (s.c.) injection of capsaicin.

Results are shown in Figure 2.

### Formalin test

In the formalin test, male ddY mice (n=16) (Japan SLC, Hamamatsu,Japan) were placed into a transparent cage (22.0 cm × 15.0 cm × 12.5 cm high) which also served as an observation chamber and were allowed to adapt to their environment for 1 h before testing. After this period, plantar subcutaneous injection of 20 µL formalin (2% in saline) using a microsyringe with 26-gauge needle. BEO and control have been administered 10 min before the injection of formalin. Each mouse was immediately returned to the observation chamber after injection. The recording of the first response (first phase) started immediately and lasted for 10 min (0-10 min). The recording of the late response (late phase) started 10 min after formalin injection and lasted for 20 min (10-30 min). In both phases, licking and biting of the injected hindpaw were defined as a nociceptive response and the total time (s) of the response was measured with a hand-held stop-watch.

Results are shown in Figure 3.

### PSNL (Partial Sciatic Nerve Ligation)test

Male ddY mice (n=20) (Kyudo Industries, Kumamoto, Japan) were anesthetized by pentobarbital anesthesia (50 mg/kg, i.p.) following the methods of Malmberg and Basbaum (1998) (Malmberg & Basbaum, 1998). BEO and control have been administered on post-operative day 7. The sciatic nerve of the right hindlimb was exposed at high thigh level through a small incision and the distal one third to one half of the dorsal portion of the sciatic nerve was tied with non-absorbable silk thread. The wound was closed with silk thread suture and covered with antibiotic powder. Immediately following surgery, the animals were kept in a soft bag cage with some food inside so that they could feed themselves without having difficulty standing. The wound healed within 1 to 2 days, and the mice with ligated nerves did not present signs of foot clonus or autotomy, but behaved normally.

The presence of mechanical allodynia (sensitization) was determined by an electronic version of the von Frey test (dynamic plantar anesthesiometer, model 37400; Ugo Basile, Milan, Italy). Animals were allowed to habituate to the testing environment and Plexiglass observation chamber (11.0 × 17.0 × 14.0 cm, length × width × height) with a wire mesh floor for approximately 1 h prior to testing. Through the wire mesh floor of the chamber, a servo-controlled mechanical stimulus (a pointed metallic filament) was applied to the midplantar region of the hindpaw. When a trial is initiated, the device raises the filament to touch the foot and progressively increases force until the animal withdraws its foot. The electro-von Frey device is capable of applying a progressively increasing punctate pressure, reaching up to 3.0 g within 5.0 s. The pressure evoking a clear voluntary hindpaw withdrawal response (usually close to 3.0 g) was recorded automatically and taken as the mechanical threshold index. In the time course experiment of PSNL-induced allodynia, baseline responses to mechanical stimulation were obtained before injury and from day 1 to day 35 post-PSNL injury. The effect of NDS BEO-BF or empty NDS on mechanical hypersensitivity was measured 7 days after the surgery when allodynia is fully developed and the pressure applied was 4.0 g. Mechanical paw withdrawal thresholds were evaluated before and after i.pl. injection. Each paw was tested three times to yield a mean value. All the testing was performed by a blind observer.

Results are shown in Figure 4.

### Itch test

The effects of NDS BEO-BF cream on scratching behaviour induced by 4-methyl-histamine administration were studied in the ddY mice (Shizuoka Laboratory Center, Japan). Groups of mice (n=8 per group) were pretreated with NDS BEO-BF cream (1.0 mg/mice) or control cream (1.0 mg/mice) for transdermal application 30 min prior or immediately before intradermal (i.d.) administration of 4 methyl-histamine (200µg/µl) and the scratching behaviour filmed for 30 min and measured offline by an independent observer. The 4-methyl-histamine is a pharmacological tool used to induce itching behaviour in mice. Latency time is of 10 minutes. It has been observed the occurrence of scratching behaviour in the mice subjected only to 4-methyl-histamine administration. This behaviour, more intense in the first minute of observation, but occurring also from 2 min and 40 seconds, was completely prevented by the topical application of the nanocream delivery system (NDS) based on α-TFS-SLN containing BEO-BF.

Results are shown in Figure 5.

### EXAMPLE 3: Formulation according to the present invention and method of preparation thereof

- Suspension of SLN in purified water FU g 84.75
- sweet almond oil g 10
- butylhydroxytoluene (BHT) 0.05 g
- sepigel g 5 (polyacrylamide 40%, C13-14 isoparaffin 20%, 7-laurate 5%, purified water FU 35%)
- methyl 4-hydroxybenzoate 0.18 g
- propyl 4-hydroxybenzoate 0.02 g

The pharmaceutical preparation requires the initial nipagine solubilization by magnetic stirrer at a temperature of 60 °C, in a quantity of water equal to half of the acqueous phase (39.88 g) present in the formulation. After nipagine solution cooling, the SLN suspension (5ml) and the rest of the purified water were added to obtain the final acqueous phase weight (84.75 g).

Solubilizing the BHT in almond oil, pouring the previously prepared solution with a quantity more or less equal to the quantity of oil, adding sepigel and stirring with a glass rod. Continuing with a geometric dilution until all the aqueous suspension has been used up. The emulsion thus obtained is processed in citounguetor for 2 minutes at speed 2. This method has the following advantages:
- homogeneously incorporating SLNs without the use of a turboemulsifier which could vary the volume and/or damage the nanoparticles;
- not changing pH of the initial solution containing SLN;
- Forming a creamy base in cold conditions, a fundamental point for particles integrity.

On the basis of the previous points the possibility of creams and gels whose preparation is closely related to temperature and heat has been ruled out.

Another method can be the suspension of SLNs in cold conditions in a basic Pharmacopoeia cream such as cetomacrogol, with the limit of nanoparticle dispersion that may not be homogeneous due to the failure to use a turbine.

### References

World Health Organization (WHO) (2012). Dementia: A Public Health Priority. Geneva: WHO.

Achterberg WP, Pieper MJ, van Dalen-Kok AH, de Waal MW, Husebo BS, Lautenbacher S, et al. (2013). Pain management in patients with dementia. Clinical interventions in aging 8: 1471-1482.

Arner S, Meyerson BA (1988). Lack of analgesic effect of opioids on neuropathic and idiopathic forms of pain. Pain 33: 11-23.

Ashwood-Smith MJ, Poulton GA, Barker M, Mildenberger M (1980). 5-Methoxypsoralen, an ingredient in several suntan preparations, has lethal, mutagenic and clastogenic properties. Nature 285: 407-409.

Bagetta G, Morrone LA, Rombola L, Amantea D, Russo R, Berliocchi L, et al. (2010). Neuropharmacology of the essential oil of bergamot. Fitoterapia 81: 453-461.

Ballard C, Howard R (2006). Neuroleptic drugs in dementia: benefits and harm. Nature reviews. Neuroscience 7: 492-500.

Ballard C, Corbett A (2013). Agitation and aggression in people with Alzheimer's disease. Current opinion in psychiatry 26: 252-259.

Ballard CG, Gauthier S, Cummings JL, Brodaty H, Grossberg GT, Robert P, et al. (2009). Management of agitation and aggression associated with Alzheimer disease. Nature reviews. Neurology 5: 245-255.

Barocelli E, Calcina F, Chiavarini M, Impicciatore M, Bruni R, Bianchi A, et al. (2004). Antinociceptive and gastroprotective effects of inhaled and orally administered Lavandula hybrida Reverchon "Grosso" essential oil. Life sciences 76: 213-223.

Berliocchi L, Russo R, Maiaru M, Levato A, Bagetta G, Corasaniti MT (2011). Autophagy impairment in a mouse model of neuropathic pain. Molecular pain 7: 83.

Berliocchi L, Maiaru M, Varano GP, Russo R, Corasaniti MT, Bagetta G, et al. (2015). Spinal autophagy is differently modulated in distinct mouse models of neuropathic pain. Molecular pain 11: 3.

Bernards MA, Lewis NG (1992). Alkyl ferulates in wound healing potato tubers. Phytochemistry 31: 3409-3412.

Boddeke HW, Best R, Boeijinga PH (1997). Synchronous 20 Hz rhythmic activity in hippocampal networks induced by activation of metabotropic glutamate receptors in vitro. Neuroscience 76: 653-658.

Braak H, Braak E (1991). Neuropathological stageing of Alzheimer-related changes. Acta neuropathologica 82: 239-259.

Burns A, Jacoby R, Levy R (1990a). Psychiatric phenomena in Alzheimer's disease. I: Disorders of thought content. The British journal of psychiatry : the journal of mental science 157: 72-76, 92-74.

Burns A, Jacoby R, Levy R (1990b). Psychiatric phenomena in Alzheimer's disease. II: Disorders of perception. The British journal of psychiatry : the journal of mental science 157: 76-81, 92-74.

Burns A, Jacoby R, Levy R (1990c). Psychiatric phenomena in Alzheimer's disease. III: Disorders of mood. The British journal of psychiatry : the journal of mental science 157: 81-86, 92-84.

Burns A, Jacoby R, Levy R (1990d). Psychiatric phenomena in Alzheimer's disease. IV: Disorders of behaviour. The British journal of psychiatry : the journal of mental science 157: 86-94.

Carnat AP, Carnat A, Fraisse D, Lamaison JL (1998). The aromatic and polyphenolic composition of lemon balm (Melissa Officinalis L. subsp. Officinalis) tea. Pharm Acta Helvetiae 72: 301-305.

Chang K.-M. and Shen C.-W. Aromatherapy Benefits Autonomic Nervous System Regulation for Elementary School Faculty in Taiwan. Evidence-Based Complementary and Alternative Medicine, vol. 2011, Article ID 946537, 7 pages, 2011.

Cho I, Kim YD (1997) Synthesis and properties of tocopherol-containing polymeric vesicle system. Macromol. Symp. 118: 631-640.

Corbett A, Husebo B, Malcangio M, Staniland A, Cohen-Mansfield J, Aarsland D, et al. (2012). Assessment and treatment of pain in people with dementia. Nature reviews. Neurology 8: 264-274.

Costa R, Dugo P, Navarra M, Raymo V, Dugo G, Mondello L. Study on the chemical composition variability of some processed bergamot (Citrus bergamia) essential oils. Flavour and Fragrance Journal 2010, 25(1), pp. 4-12.

Dastmalchi K, Ollilainen V, Lackman P, Boije af Gennas G, Dorman HJ, Jarvinen PP, et al. (2009). Acetylcholinesterase inhibitory guided fractionation of Melissa officinalis L. Bioorganic & medicinal chemistry 17: 867-871.

Dugo P, Mondello L, Dugo L, Stancanelli R, Dugo G (2000). LC-MS for the identification of oxygen heterocyclic compounds in citrus essential oils. J Pharm Biomed Anal 24: 147-150.

Fang T, Goto M, Sasaki M, and Hirose T. Combination of supercritical CO2 and vacuum distillation for the fractionation of bergamot oil. J Agric Food Chem 2004, 52:5162-5167.

Focà, A., Liberto, M.C. (2015). Historical Notes on Essential Oils and Aromatherapy with Special Reference to Bergamot Essential Oil. In: "Aromatherapy: Basic Mechanisms and Evidence Based Clinical Use". G. Bagetta, M. Cosentino, T. Sakurada, Eds. CRC Press, pp. 1-16.

Gold G, Bouras C, Kovari E, Canuto A, Glaria BG, Malky A, et al. (2000). Clinical validity of Braak neuropathological staging in the oldest-old. Acta neuropathologica 99: 579-582; discussion 583-574.

Graham PH, Browne L, Cox H, and Graham J. Inhalation aromatherapy during radiotherapy: results of a placebo-controlled double-blind randomized trial. Journal of Clinical Oncology, vol. 21, no. 12, pp. 2372-2376, 2003.

Gasco MR (1993) US Patent 5250236.

Hohmann J, Zupko I, Redei D, Csanyi M, Falkay G, Mathe I, et al. (1999). Protective effects of the aerial parts of Salvia officinalis, Melissa Officinalis and Lavandula angustifolia and their constituents against enzyme-dependent and enzyme-independent lipid peroxidation. Planta medica 65: 576-578.

Husebo BS, Ballard C, Sandvik R, Nilsen OB, Aarsland D (2011). Efficacy of treating pain to reduce behavioural disturbances in residents of nursing homes with dementia: cluster randomised clinical trial. Bmj 343: d4065.

Jimbo D, Kimura Y, Taniguchi M, Inoue M, Urakami K (2009). Effect of aromatherapy on patients with Alzheimer's disease. Psychogeriatrics : the official journal of the Japanese Psychogeriatric Society 9: 173-179.

Katsuyama S, Otowa A, Kamio S, Sato K, Yagi T, Kishikawa Y, et al. (2015). Effect of plantar subcutaneous administration of bergamot essential oil and linalool on formalin-induced nociceptive behavior in mice. Biomedical research 36: 47-54.

Kawanishi K, Yasufuku J, Ishikawa A, Ashimoto Y (1990) Long-chain alkyl ferulates in three varieties of Ipomoea batatas (L.) Lam. J. Agric. Food Chem. 38: 105-108.

Kennedy DO, Wake G, Savelev S, Tildesley NT, Perry EK, Wesnes KA, et al. (2003). Modulation of mood and cognitive performance following acute administration of single doses of Melissa officinalis (Lemon balm) with human CNS nicotinic and muscarinic receptor-binding properties. Neuropsychopharmacology : official publication of the American College of Neuropsychopharmacology 28: 1871-1881.

Koppel DE (1972) Analysis of Macromolecular Polydispersity in Intensity Correlation Spectroscopy: The Method of Cumulants. J. Chem. Phys. 57: 4814

Kuwahata H, Komatsu T, Katsuyama S, Corasaniti MT, Bagetta G, Sakurada S, et al. (2013). Peripherally injected linalool and bergamot essential oil attenuate mechanical allodynia via inhibiting spinal ERK phosphorylation. Pharmacology, biochemistry, and behavior 103: 735-741.

Lyketsos CG, Steinberg M, Tschanz JT, Norton MC, Steffens DC, Breitner JC (2000). Mental and behavioral disturbances in dementia: findings from the Cache County Study on Memory in Aging. The American journal of psychiatry 157: 708-714.

Malmberg AB, Basbaum AI (1998). Partial sciatic nerve injury in the mouse as a model of neuropathic pain: behavioral and neuroanatomical correlates. Pain 76: 215-222.

Manns ID, Alonso A, Jones BE (2003). Rhythmically discharging basal forebrain units comprise cholinergic, GABAergic, and putative glutamatergic cells. Journal of neurophysiology 89: 1057-1066.

Margallo-Lana M, Swann A, O'Brien J, Fairbairn A, Reichelt K, Potkins D, et al. (2001). Prevalence and pharmacological management of behavioural and psychological symptoms amongst dementia sufferers living in care environments. International journal of geriatric psychiatry 16: 39-44.

Mondello L, Stagno d'Alcontres I, Del Duce R, Crispo F (1993). On the genuineness of citrus essential oils. Part XL. The composition of the coumarins and psoralens of calabrian bergamot essential oil (Citrus bergamia Risso). Flav Fragr J 8: 17-24.

Morrone LA, Scuteri D, Rombola L, Mizoguchi H, Bagetta G (2017). Opioids Resistance in Chronic Pain Management. Current neuropharmacology 15: 444-456.

Morrone LA, Rombola L, Pelle C, Corasaniti MT, Zappettini S, Paudice P, et al. (2007). The essential oil of bergamot enhances the levels of amino acid neurotransmitters in the hippocampus of rat: implication of monoterpene hydrocarbons. Pharmacological research 55: 255-262.

Müller RH and Lucks J.S (1996) EP 0605497 (1996). Ndao, D.H., Ladas, E.J., Cheng, B., Sands, S.A., Snyder, K.T., Garvin Jr., J.H., Kelly, K.M. Inhalation aromatherapy in children and adolescents undergoing stem cell infusion: results of a placebo-controlled double-blind trial. Psycho-Oncology, vol. 21, no. 3, pp. 247-254, 2012.

Ni CH, Hou WH, Kao CC, Chang ML, Yu LF, Wu CC, Chen C. The Anxiolytic Effect of Aromatherapy on Patients Awaiting Ambulatory Surgery: a Randomized Controlled Trial. Evid Based Complement Alternat Med. 2013; 2013:927419.

Ortiz C, Vazquez B, San Roman J (1999) Hydrophilic acrylic biomaterials derived from vitamin E with antioxidant properties. J. Biomed. Mater. Res. 45: 184.

Perry EK, Pickering AT, Wang WW, Houghton P, Perry NS (1998). Medicinal plants and Alzheimer's disease: Integrating ethnobotanical and contemporary scientific evidence. Journal of alternative and complementary medicine 4: 419-428.

Perry EK, Pickering AT, Wang WW, Houghton PJ, Perry NS (1999). Medicinal plants and Alzheimer's disease: from ethnobotany to phytotherapy. The Journal of pharmacy and pharmacology 51: 527-534.

Petersen M, Simmonds MS (2003). Rosmarinic acid. Phytochemistry 62: 121-125.

Rapisarda, A., Germanò, M.P. (2013). Citrus × bergamia Risso & Poiteau - Botanical Classification, Morphology, and Anatomy. In: "Citrus Bergamia: Bergamot and its Derivatives", G. Dugo, I. Bonaccorsi, Eds. CRC Press, Boca Raton, pp. 9-24.

Reverchon E, lacuzio G. Supercritical desorption of bergamot peel oil from silica gel Experiments and mathematical modelling. Chem Engin Sci 1997, 52:3553-3559.

Rombola L, Corasaniti MT, Rotiroti D, Tassorelli C, Sakurada S, Bagetta G, et al. (2009). Effects of systemic administration of the essential oil of bergamot (BEO) on gross behaviour and EEG power spectra recorded from the rat hippocampus and cerebral cortex. Functional neurology 24: 107-112.

Russo R, Cassiano MG, Ciociaro A, Adornetto A, Varano GP, Chiappini C, et al. (2014). Role of D-Limonene in autophagy induced by bergamot essential oil in SH-SY5Y neuroblastoma cells. PloS one 9: e113682.

Sakurada T, Katsumata K, Tan-No K, Sakurada S, Kisara K (1992). The capsaicin test in mice for evaluating tachykinin antagonists in the spinal cord. Neuropharmacology 31: 1279-1285.

Sakurada T, Mizoguchi H, Kuwahata H, Katsuyama S, Komatsu T, Morrone LA, et al. (2011). Intraplantar injection of bergamot essential oil induces peripheral antinociception mediated by opioid mechanism. Pharmacology, biochemistry, and behavior 97: 436-443.

Savva GM, Zaccai J, Matthews FE, Davidson JE, McKeith I, Brayne C, et al. (2009). Prevalence, correlates and course of behavioural and psychological symptoms of dementia in the population. The British journal of psychiatry : the journal of mental science 194: 212-219.

Schneider LS, Dagerman K, Insel PS (2006). Efficacy and adverse effects of atypical antipsychotics for dementia: meta-analysis of randomized, placebo-controlled trials. The American journal of geriatric psychiatry : official journal of the American Association for Geriatric Psychiatry 14: 191-210.

Scuteri D, Morrone LA, Rombola L, Avato PR, Bilia AR, Corasaniti MT, et al. (2017). Aromatherapy and Aromatic Plants for the Treatment of Behavioural and Psychological Symptoms of Dementia in Patients with Alzheimer's Disease: Clinical Evidence and Possible Mechanisms. Evidence-based complementary and alternative medicine : eCAM 2017: 9416305.

Taniguchi H, Nomura E, Tsuno T, Minami S (1998) Eur. Patent. Appl. 0681825 A2
Tariot PN, Mack JL, Patterson MB, Edland SD, Weiner MF, Fillenbaum G, et al. (1995). The Behavior Rating Scale for Dementia of the Consortium to Establish a Registry for Alzheimer's Disease. The Behavioral Pathology Committee of the Consortium to Establish a Registry for Alzheimer's Disease. The American journal of psychiatry 152: 1349-1357.

Trombino S, Cassano R, Muzzalupo R, Pingitore A, Cione E, Picci N (2009) Stearyl ferulate-based solid lipid nanoparticle for the encapsulation and stabilization of beta-carotene and alpha tocopherol. Surf. B, Biointerf. 72:181-187.

Verzera A, Lamonica G, Mondello L, Trozzi A, Dugo G (1996). The composition of bergamot oil. Perfum Flavor 21: 19-34.

Wake G, Court J, Pickering A, Lewis R, Wilkins R, Perry E (2000). CNS acetylcholine receptor activity in European medicinal plants traditionally used to improve failing memory. Journal of ethnopharmacology 69: 105-114.

Yu WH, Cuervo AM, Kumar A, Peterhoff CM, Schmidt SD, Lee JH, et al. (2005). Macroautophagy--a novel Beta-amyloid peptide-generating pathway activated in Alzheimer's disease. J Cell Biol 171: 87-98.

Zaynoun ST, Johnson BE, Frain-Bell W (1977). A study of oil of bergamot and its importance as a phototoxic agent. I. Characterization and quantification of the photoactive component. Br J Dermatol 96: 475-482.

## Claims

1. Solid lipid nanoparticles, wherein said nanoparticles are α-tocopheryl stearate based and comprise bergamot essential oil free of psoralens.

2. Solid lipid nanoparticles according to claim 1, wherein said solid lipid nanoparticles have a size of 444.03nm ± 60.07.

3. Solid lipid nanoparticles according to anyone of claims 1-2, wherein said bergamot essential oil comprises limonene, linalool and linalyl acetate.

4. Pharmaceutical composition comprising solid lipid nanoparticles as defined in any one of claims 1-3, as active principle, in association with one or more excipients and/or adjuvants.

5. Pharmaceutical composition according to claim 4, said pharmaceutical composition being in the form of a cream or gel.

6. Solid lipid nanoparticles according to any one of claims 1-3 or pharmaceutical composition according to any one of claims 4-5, for use as a medicament.

7. Solid lipid nanoparticles according to any one of claims 1-3 or pharmaceutical composition according to any one of claims 4-5, for use in the treatment of acute and chronic pain, such as the neuropathic type of pain or pain occurring in the course of cancer or pain occurring in the course of chronic neurodegenerative diseases, such as Parkinson's disease and Alzheimer's disease.

8. Solid lipid nanoparticles according to any one of claims 1-3 or pharmaceutical composition according to any one of claims 4-5, for use in the prevention or treatment of behavioural and psychiatric symptoms of dementia, or of behavioural or mood disturbances emerging from stressful conditions, such as itch.

9. Method for obtaining solid lipid nanoparticles according to any one of claims 1-3, said method comprising or consisting of:
a) mixing α-tocopheryl stearate in the presence of bergamot essential oil by heating, for example bergamot essential oil in a percentage of 10% in comparison to α-tocopheryl stearate; b) adding a water solution of a one or more emulsifiers, such as polysorbate 20, polysorbate 60, soy phosphatidylcholine, and sodium taurodeoxycholate, preferably sodium taurodeoxycholate, one or more co-emulsifiers, such as sodium monooctylphosphate, n-butanol, preferably butanol, water, in order to obtain a microemulsion; c) dispersing the microemulsion in cold water under agitation, preferably in a ratio microemulsion:cold water of 1:20.

## Patentansprüche

1. Feste Lipidnanopartikel, wobei die Nanopartikel auf α-Tocopherylstearat basieren und bergamottöl enthalten, das frei von Psoralenen ist.

2. Feste Lipidnanopartikel nach Anspruch 1, wobei die festen Lipidnanopartikel eine Größe von 444,03nm ± 60,07 aufweisen.

3. Feste Lipidnanopartikel nach einem der Ansprüche 1-2, wobei das ätherische Bergamotteöl Limonen, Linalool und Linalylacetat enthält.

4. Pharmazeutische Zusammensetzung, die feste Lipidnanopartikel gemäß einem der Ansprüche 1-3 als Wirkstoff in Verbindung mit einem oder mehreren Hilfsstoffen und/oder Adjuvanzien enthält.

5. Pharmazeutische Zusammensetzung nach Anspruch 4, wobei die pharmazeutische Zusammensetzung in Form einer Creme oder eines Gels vorliegt.

6. Feste Lipidnanopartikel gemäß einem der Ansprüche 1-3 oder eine pharmazeutische Zusammensetzung gemäß einem der Ansprüche 4-5 zur Verwendung als Arzneimittel.

7. Feste Lipidnanopartikel gemäß einem der Ansprüche 1-3 oder eine pharmazeutische Zusammensetzung gemäß einem der Ansprüche 4-5 zur Verwendung bei der Behandlung von akuten und chronischen Schmerzen, wie beispielsweise neuropathischen Schmerzen oder Schmerzen, die im Verlauf von Krebserkrankungen auftreten, oder Schmerzen, die im Verlauf chronischer neurodegenerativer Erkrankungen wie der Parkinson-Krankheit und der Alzheimer-Krankheit auftreten.

8. Feste Lipidnanopartikel gemäß einem der Ansprüche 1-3 oder eine pharmazeutische Zusammensetzung gemäß einem der Ansprüche 4-5 zur Verwendung bei der Vorbeugung oder Behandlung von Verhaltens- und psychiatrischen Symptomen der Demenz oder von Verhaltens- oder Stimmungsstörungen, die aus Stresssituationen wie beispielsweise Juckreiz resultieren.

9. Verfahren zur Herstellung fester Lipidnanopartikel gemäß einem der Ansprüche 1-3, wobei das Verfahren umfasst oder darin besteht:
a) Vermischen von α-Tocopherylstearat mit ätherischem Bergamotteöl durch Erhitzen, beispielsweise mit einem Anteil von 10% ätherischem Bergamotteöl im Verhältnis zu α-Tocopherylstearat; b) Zugabe einer wässrigen Lösung eines oder mehrerer Emulgatoren, wie Polysorbat 20, Polysorbat 60, Soja-Phosphatidylcholin und Natriumtaurodeoxycholat, vorzugsweise Natriumtaurodeoxycholat, eines oder mehrerer Co-Emulgatoren, wie Natriummonooctylphosphat, n-Butanol, vorzugsweise Butanol, sowie Wasser, um eine Mikroemulsion zu erhalten; c) Dispergieren der Mikroemulsion in kaltem Wasser unter Rühren, vorzugsweise in einem Verhältnis von Mikroemulsion zu kaltem Wasser von 1:20.

## Revendications

1. Nanoparticules lipidiques solides, lesdites nanoparticules étant à base de stéarate d'α-tocophéryle et contenant de l'huile essentielle de bergamote exempte de psoralènes.

2. Nanoparticules lipidiques solides selon la revendication 1, **caractérisées en ce que** lesdites nanoparticules lipidiques solides ont une taille de 444,03nm ± 60,07.

3. Nanoparticules lipidiques solides selon l'une quelconque des revendications 1-2, dans lesquelles ladite huile essentielle de bergamote comprend du limonène, du linalol et de l'acétate de linalyle.

4. Composition pharmaceutique comprenant, en tant que principe actif, des nanoparticules lipidiques solides telles que définies dans l'une quelconque des revendications 1-3, en association avec un ou plusieurs excipients et/ou adjuvants.

5. Composition pharmaceutique selon la revendication 4, ladite composition pharmaceutique se présentant sous la forme d'une crème ou d'un gel.

6. Nanoparticules lipidiques solides selon l'une quelconque des revendications 1-3 ou composition pharmaceutique selon l'une quelconque des revendications 4-5, destinées à être utilisées comme médicament.

7. Nanoparticules lipidiques solides selon l'une quelconque des revendications 1-3 ou composition pharmaceutique selon l'une quelconque des revendications 4-5, destinées à être utilisées dans le traitement de la douleur aiguë et chronique, telle que la douleur de type neuropathique, la douleur survenant dans le cadre d'un cancer ou la douleur survenant dans le cadre de maladies neurodégénératives chroniques, telles que la maladie de Parkinson et la maladie d'Alzheimer.

8. Nanoparticules lipidiques solides selon l'une quelconque des revendications 1-3 ou composition pharmaceutique selon l'une quelconque des revendications 4-5, destinées à être utilisées dans la prévention ou le traitement des symptômes comportementaux et psychiatriques de la démence, ou des troubles comportementaux ou de l'humeur résultant de situations stressantes, telles que les démangeaisons.

9. Procédé d'obtention de nanoparticules lipidiques solides selon l'une quelconque des revendications 1-3, ledit procédé comprenant ou consistant en:
a) mélanger du stéarate d'a-tocophéryle en présence d'huile essentielle de bergamote par chauffage, par exemple de l'huile essentielle de bergamote à raison de 10% par rapport au stéarate d'a-tocophéryle; b) ajouter une solution aqueuse d'un ou plusieurs émulsifiants, tels que le polysorbate 20, le polysorbate 60, la phosphatidylcholine de soja et le taurodésoxycholate de sodium, de préférence le taurodésoxycholate de sodium, un ou plusieurs co-émulsifiants, tels que le monooctylphosphate de sodium, le n-butanol, de préférence le butanol, et de l'eau, afin d'obtenir une microémulsion; c) disperser la microémulsion dans de l'eau froide sous agitation, de préférence dans un rapport microémulsion:eau froide de 1:20.
